(19) Europäisches Patentamt
European Patent Office
Office européen des brevets

(11) **EP 3 970 803 A1**

(12) **EUROPEAN PATENT APPLICATION**

(43) Date of publication:
**23.03.2022 Bulletin 2022/12**

(21) Application number: **20197603.2**

(22) Date of filing: **22.09.2020**

(51) International Patent Classification (IPC):
*A61Q 19/00* (2006.01)    *A61P 17/00* (2006.01)
*A61P 17/10* (2006.01)    *A61K 8/34* (2006.01)
*A61K 8/365* (2006.01)    *A61K 8/19* (2006.01)
*A61K 8/81* (2006.01)    *A61K 8/73* (2006.01)
*A61K 8/86* (2006.01)

(52) Cooperative Patent Classification (CPC):
**A61Q 19/00; A61K 8/19; A61K 8/34; A61K 8/345;
A61K 8/365; A61K 8/735; A61K 8/8152;
A61K 8/86; A61P 17/00; A61P 17/10**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Beiersdorf AG
20253 Hamburg (DE)**

(72) Inventor: **The designation of the inventor has not
yet been filed**

(74) Representative: **Uexküll & Stolberg
Partnerschaft von
Patent- und Rechtsanwälten mbB
Beselerstraße 4
22607 Hamburg (DE)**

(54) **COSMETIC COMPOSITION OF LOW OSMOLALITY**

(57) The present invention generally relates to the field of skin care. More particularly, the invention relates to a skin care composition that has a particularly low osmolality. The skin care composition of the invention therefore is specifically suitable for supporting the skin microbiome, because it does not create an osmotic stress that would be harmful to the bacteria after its topical application to the skin. Accordingly, skin care composition of the invention preserves and improves the vitality of the bacteria of the skin microbiome, which enhances the stability of a healthy skin microbiome. The skin care composition of the present invention can be thus used for treating or preventing skin diseases, for promoting the development of a healthy skin microbiome, for stabilizing a healthy skin microbiome, and for maintaining or promoting the vitality of bacteria of the skin microbiome.

Figure 2

| B | reduced Hydrogel | | |
|---|---|---|---|
| | t0(5min ) | t 1,5h | t 3,0h |
| forearm + formula | 48 | 52 | 38 |
| C. Acnes bouillon + | 100 | 103 | 96 |

EP 3 970 803 A1

Printed by Jouve, 75001 PARIS (FR)

**Description**

The present invention generally relates to the field of skin care. More particularly, the invention relates to a skin care composition that has a particularly low osmolality. The skin care composition of the invention therefore is specifically suitable for supporting the skin microbiome, because it does not create an osmotic stress that would be harmful to the bacteria after its topical application to the skin. Accordingly, skin care composition of the invention preserves and improves the vitality of the bacteria of the skin microbiome, which enhances the stability of a healthy skin microbiome. The skin care composition of the present invention can be thus used for treating or preventing skin diseases, for promoting the development of a healthy skin microbiome, for stabilizing a healthy skin microbiome, and for maintaining or promoting the vitality of bacteria of the skin microbiome.

## BACKGROUND OF THE INVENTION

The human skin is colonized by millions of microorganisms, in particular bacteria and fungi. Most of these microorganisms are harmless or even beneficial to their host. The entirety of the microorganisms living on the human skin is referred to as the "skin microbiome". There is emerging evidence that the skin microbiome has a direct influence on human health. For example, bacteria of the skin microbiome contribute to health by secreting antimicrobial compounds that provide a barrier to infection of the body with pathogenic bacteria or fungi. In addition, the skin microbiome interacts with the immune system and effectively stimulates the immune system to combat pathogenic bacteria and fungi.

The human skin is a habitat that is generally unfavorable for microorganisms. The skin is salty, dry and continuously exposed to UV radiation. In addition, the availability of nutrients on the skin surface is limited to compounds that are released from dead skin cells of the upper epidermal layer. Due to these harsh conditions, the overall number of bacterial and fungal cells on skin is rather low, especially in dry areas such as the forearm, leg and lower back. Daily washing the skin with soap as well as the use of inappropiate cosmetic products provide additional antimicrobial effects which further reduce the overall number of bacterial and fungal cells on skin and may result in a disturbance of the skin microbiome.

In a healthy microbiome, there appears to be a balance of the resident microorganisms with respect to each other. A disturbance of said balance is thought to contribute to diseases or disorders, such as acne or eczema. In particular, if the number of bacteria from the resident flora, such as *Staphylococcus epidermidis* and *Cutitbacterium acnes,* becomes too low, there is a risk that the skin is colonized with pathogenic or potentially pathogenic bacteria, such as *Staphylococcus aureus.* A healthy and stable microbiome normally prevents the colonization of the skin with such pathogenic or potentially pathogenic bacteria. Accordingly, there is a need for means and methods that result in the maintainance of a healthy skin microbiome. In addition, there is a need for means and methods that could maintain or promote the vitality of the resident microorganisms in the microbiome.

## DESCRIPTION OF THE INVENTION

It has been found in the course of the present invention that it is possible to actively support a healthy and stable microbiome by application of a skin care composition that has a particularly low osmolality. Specifically, it has been found that commonly used skin care compositions regularly comprise a high amount of components and additives that significantly increase the number of osmotically active solutes in the compositions. Such solutes lead to a high level of osmotic stress after application of the composition to the skin. This holds particularly true for water-containing skin care compositions, such as gels. After application to the skin the water contained in the composition evaporates to a significant extent so that the remaining water on the skin surface comprises salts and other osmotically active components in highly concentrated form. These concentrated compounds are able to bind free water in the environment which is crucial for the bacterial metabolism and hence for viability of the bacteria. In addition, due to the hyperosmotic conditions on the skin surface, water from inside of the bacterial cells diffuses along the osmotic gradient to the outside. This severely impacts viability of the bacterial cells.

The present invention is based on the insight that by reduction of amounts or even a complete omission of certain commonly used components, compositions and formulations with a particularly low osmolality can be obtained which are significantly less harmful to the bacteria that occur on the skin surface. As a consequence, such compositions and formulations are particularly suitable for maintaining or enhancing the vitality of bacteria that occur on the skin, preferably the human skin.

Accordingly, in a first aspect, the present invention relates to a skin care composition for maintaining or promoting the vitality of bacteria on the skin surface, wherein said composition

    (a) is a water-containing composition which is formulated for topical application to the skin, and

(b) has an osmolality of 4000 mOsm/kg water or less.

The osmolality of a solution quantifies the osmotically active particles or ions contained in a solution. In contrast to molarity, which determines the number of particles or ions in a volume of fluid, osmolality refers to the mass weight. It is normally expressed as mOsm/kg water or mmol/kg water. The actual osmolality of a composition can be determined in accordance with methods well-known in the art. For example, osmolality can be measured by using a freezing point depression osmometer or a vapour pressure depression osmometer. Since the freezing point of a water-based composition is depressed dependent on the number of osmotically active particles or ions contained, it is possible to measure osmolality by determining the freezing point of the composition.

Since measuring the actual osmolality is cumbersome, the osmolality of a composition is often determined in practice by calculation the "theoretical osmolality" based on the molar masses of the compounds contained in said composition and the amounts of the compounds in the composition. As used herein, the theoretical osmolality $b_{osm}$ indicates the molality of the osmotically active particles in a solution. It is calculated according to the formula:

$$b_{osm} = \frac{n_{osm}}{m_{Solvent}}$$

wherein $n_{osm}$ indicates the amount of osmotically active particles in moles and $m_{Solvent}$ indicates the mass of the solvent in kg.

According to the invention, the skin care composition has a theoretical osmolality of 4000 mOsm/kg water or less. Preferably, the composition has a theoretical osmolality of 3900 mOsm/kg water or less, 3800 mOsm/kg water or less, 3700 mOsm/kg water or less, 3600 mOsm/kg water or less, 3500 mOsm/kg water or less, 3400 mOsm/kg water or less, 3300 mOsm/kg water or less, 3200 mOsm/kg water or less, 3100 mOsm/kg water or less, or 3000 mOsm/kg water or less. Stated differently, it is particularly preferred that skin care composition has a theoretical osmolality in the range between 3000-4000 mOsm/kg water, more preferably between 3100-3900 mOsm/kg water, between 3200-3800 mOsm/kg water, between 3300-3700 mOsm/kg water, or between 3400-3600 mOsm/kg water.

The composition of the invention is a water-containing composition. The water content of the composition is 30% (w/w) water or more. This means that the composition of the invention comprises 30% (w/w) water or more, 35% (w/w) water or more, 40% (w/w) water or more, 45% (w/w) water or more, 50% (w/w) water or more, 55% (w/w) water or more, 60% (w/w) water or more, 65% (w/w) water or more, 70% (w/w) water or more, 75% (w/w) water or more, 80% (w/w) water or more, or 85% (w/w) water or more. It is particularly preferred that the composition of the invention comprises at least 70% (w/w) or at least 75% (w/w) water.

The skin care composition of the present invention is preferably formulated as a ready-to-use composition which is suitable for direct topical administration to the skin. Such a composition may be provided in different forms, including, but not limited to, in the form of a gel, lotion, fluid, cream, ointment, solution, a water-based emulsion, or the like. In a particularly preferred embodiment, the skin care composition of the present invention is formulated as a gel or lotion. Alternatively, the skin care compositions of the present invention may be provided as a kit-of-parts in which certain components of the compositions are provided spatially separated from the other components. For example, where the composition is an emulsion, the lipophilic and the hydrophilic components may be provided spatially separated, e.g. in two separated chambers, wherein the first chamber contains the lipophilic components and the second chamber contains the hydrophilic components. After mixing the contents of both chambers, the skin care composition is ready for use. Suitable two-chamber-systems are described, for example, in WO 2019/238968.

The present skin care compositions are particularly useful for administering and establishing health-promoting bacteria on the skin of a subject, preferably a human subject. For example, skin care compositions of the invention can be applied in combination with lyophilized bacteria, as described in WO 2019/238968. The lyophilized or spray-dried bacteria, in particular S. *epidermidis* and/or C. *acnes,* are provided in the first chamber of a two-chamber system suspended in a lipid or oil. The second chamber may comprise a water-containing cosmetic composition as described herein. The content of both chambers are mixed before application to the skin.

To provide for a low level of osmolality, the skin care compositions of the invention will use only reduced amounts of compounds which, upon their dissolution in water, are osmotically active. In some embodiments, certain osmotically active compounds like NaCl, KCl, $CaCl_2$ or $MgCl_2$ are completely omitted.

In one embodiment, the amount of NaCl in the composition which is to be administered to the skin is less than 1% (w/w), less than 0.9% (w/w), less than 0.8% (w/w), less than 0.7% (w/w), less than 0.6% (w/w), less than 0.5% (w/w), less than 0.4% (w/w), less than 0.3% (w/w), less than 0.2% (w/w), or less than 0.1% (w/w). In a particularly preferred embodiment, the composition does not comprise any NaCl.

In yet one embodiment, the amount of KCl in the composition which is to be administered to the skin is less than 1% (w/w), less than 0.9% (w/w), less than 0.8% (w/w), less than 0.7% (w/w), less than 0.6% (w/w), less than 0.5% (w/w), less than 0.4% (w/w), less than 0.3% (w/w), less than 0.2% (w/w), or less than 0.1% (w/w). In a particularly preferred embodiment, the composition does not comprise any KCl.

In one embodiment, the amount of $CaCl_2$ in the composition which is to be administered to the skin is less than 1% (w/w), less than 0.9% (w/w), less than 0.8% (w/w), less than 0.7% (w/w), less than 0.6% (w/w), less than 0.5% (w/w), less than 0.4% (w/w), less than 0.3% (w/w), less than 0.2% (w/w), or less than 0.1% (w/w). In a particularly preferred embodiment, the composition does not comprise any $CaCl_2$.

In one embodiment, the amount of $MgCl_2$ in the composition which is to be administered to the skin is less than 1% (w/w), less than 0.9% (w/w), less than 0.8% (w/w), less than 0.7% (w/w), less than 0.6% (w/w), less than 0.5% (w/w), less than 0.4% (w/w), less than 0.3% (w/w), less than 0.2% (w/w), or less than 0.1% (w/w). In a particularly preferred embodiment, the composition does not comprise any $MgCl_2$.

In one embodiment, the amount of $MgCO_3$ in the composition which is to be administered to the skin is less than 1% (w/w), less than 0.9% (w/w), less than 0.8% (w/w), less than 0.7% (w/w), less than 0.6% (w/w), less than 0.5% (w/w), less than 0.4% (w/w), less than 0.3% (w/w), less than 0.2% (w/w), or less than 0.1% (w/w). In a particularly preferred embodiment, the composition does not comprise any $MgCO_3$.

In one embodiment, the amount of $MgSO_4$ in the composition which is to be administered to the skin is less than 1% (w/w), less than 0.9% (w/w), less than 0.8% (w/w), less than 0.7% (w/w), less than 0.6% (w/w), less than 0.5% (w/w), less than 0.4% (w/w), less than 0.3% (w/w), less than 0.2% (w/w), or less than 0.1% (w/w). In a particularly preferred embodiment, the composition does not comprise any $MgSO_4$.

Another component that contributes to osmolality is glycerol. It is preferred according to the invention that the amount of glycerol in the composition which is to be administered to the skin is less than 10% (w/w), less than 9% (w/w), less than 8% (w/w), less than 7% (w/w), less than 6% (w/w), less than 5% (w/w), less than 4% (w/w), less than 3% (w/w), less than 2% (w/w), or less than 1% (w/w). In a particularly preferred embodiment, the composition does not comprise any glycerol.

It is preferred that the skin care composition of the invention comprises less than 0.5% (w/w) of NaCl and less than 5% (w/w) of glycerol. In one embodiment, the composition comprises less than 0.5% (w/w) of NaCl and no glycerol. In another embodiment, the composition comprises less than 5% (w/w) of glycerol and no NaCl.

It is preferred that the skin care composition of the invention comprises less than 0.5% (w/w) of KCl and less than 5% (w/w) of glycerol. In one embodiment, the composition comprises less than 0.5% (w/w) of KCl and no glycerol. In another embodiment, the composition comprises less than 5% (w/w) of glycerol and no KCl.

It is preferred that the skin care composition of the invention comprises less than 0.5% (w/w) of $CaCl_2$ and less than 5% (w/w) of glycerol. In one embodiment, the composition comprises less than 0.5% (w/w) of $CaCl_2$ and no glycerol. In another embodiment, the composition comprises less than 5% (w/w) of glycerol and no $CaCl_2$.

It is preferred that the skin care composition of the invention comprises less than 0.5% (w/w) of $MgCl_2$ and less than 5% (w/w) of glycerol. In one embodiment, the composition comprises less than 0.5% (w/w) of $MgCl_2$ and no glycerol. In another embodiment, the composition comprises less than 5% (w/w) of glycerol and no $MgCl_2$.

Preferably, the skin care composition of the invention comprises less than less than 0.5% (w/w) aluminum stearate or less than 0.5% (w/w) magnesium stearate, respectively. More preferably, the skin care composition of the invention comprises no aluminum stearate or magnesium stearate.

Preferably, the skin care composition of the invention comprises less than less than 0.05% (w/w) citric acid or sodium citrate, respectively. More preferably, the skin care composition of the invention comprises no citric acid or sodium citrate.

A particularly preferred skin care composition of the invention has an osmolality of 4000 mOsm/kg water or less and comprises

(a) at least 50% (w/w) water,
(b) less than 0.5% (w/w) of NaCl,
(c) less than 5% (w/w) of glycerol.

Another particularly preferred skin care composition of the invention has an osmolality of 4000 mOsm/kg water or less and comprises

(a) at least 50% (w/w) water,
(b) less than 0.5% (w/w) of KCl,
(c) less than 5% (w/w) of glycerol.

Another particularly preferred skin care composition of the invention has an osmolality of 4000 mOsm/kg water or less and comprises

(a) at least 50% (w/w) water,
(b) less than 0.5% (w/w) of $CaCl_2$,
(c) less than 5% (w/w) of glycerol.

Another particularly preferred skin care composition of the invention has an osmolality of 4000 mOsm/kg water or less and comprises

(a) at least 50% (w/w) water,
(b) less than 0.5% (w/w) of $MgCl_2$,
(c) less than 5% (w/w) of glycerol.

Another particularly preferred skin care composition of the invention has an osmolality of 4000 mOsm/kg water or less and comprises

(a) at least 50% (w/w) water,
(b) less than 0.5% (w/w) of NaCl,
(c) less than 5% (w/w) of glycerol.

Another particularly preferred skin care composition of the invention has an osmolality of 4000 mOsm/kg water or less and comprises

(a) at least 50% (w/w) water,
(b) less than 0.5% (w/w) of KCl,
(c) less than 5% (w/w) of glycerol.

Another particularly preferred skin care composition of the invention has an osmolality of 4000 mOsm/kg water or less and comprises

(a) at least 50% (w/w) water,
(b) less than 0.5% (w/w) of $CaCl_2$,
(c) less than 5% (w/w) of glycerol.

Another particularly preferred skin care composition of the invention has an osmolality of 4000 mOsm/kg water or less and comprises

(a) at least 50% (w/w) water,
(b) less than 0.5% (w/w) of $MgCl_2$,
(c) less than 5% (w/w) of glycerol.

The skin care composition of the invention may further comprise compounds and additives that can be metabolized by the microorganisms of the skin microbiome, thereby promoting their growth. In particular, these compounds and additives, upon their topical application to the skin, can be used as carbon, nitrogen, or lipid source by the microorganisms of the skin microbiome. The topical application of such compounds therefore provides improved metabolic conditions for these microorganisms on the skin surface.

The growth-enhancing compounds are metabolized by the resident microorganisms, i.e. the microorganisms that normally occur as part of the human skin microbiome, much more effective compared to transient microorganisms which colonize the skin only under temporarily, e.g. after touching contaminated objects with the hands. As a result, the compounds provide a growth advantage to resident microorganisms, such as *S. epidermidis* and *C. acnes,* over pathogenic microorganisms like *S. aureus.* In this way, these compounds assist in stabilizing and promoting growth of the resident microorganisms of the skin microbiome.

Accordingly, in another embodiment, the present invention relates to a skin care composition as defined above which further comprises one or more of the following

(a) pyruvate as a carbon source,
(b) lactate as a carbon source,
(c) urea as a nitrogen source,
(d) arginine as a nitrogen source, and/or
(e) caprylic/capric triglycerides as a lipid source.

In one preferred embodiment, the composition of the invention comprises pyruvate as a carbon source. Pyruvate is able to significantly increase the cell number of the resident bacteria when applied in the form of a skin care composition to the facial skin. As used herein, pyruvate refers to a salt or ester of 2-oxopropanoic acid. Preferably, the pyruvate in the composition is calcium or sodium pyruvate. Sodium pyruvate is used in some cosmetic compositions for stimulating collagen synthesis. It is preferred that the pyruvate is present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.7 to 2.0% (w/w), more preferably 0.8 to 1.5% (w/w), such as 1.0% (w/w). In some embodiments, the amount of pyruvate present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w).

In another preferred embodiment, the composition of the invention comprises lactate as a carbon source. Like pyruvate, lactate is able to increase the cell number of the resident bacteria when applied to the facial skin. As used herein, lactate refers to a salt or ester of lactic acid. Preferably, the lactate in the composition is calcium or sodium lactate. Sodium lactate is used as a humectant in some cosmetic compositions as well as for pH stabilization. It is preferred that the lactate is present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.7 to 2.0% (w/w), more preferably 0.8 to 1.5% (w/w), such as 1.0% (w/w). In some embodiments, the amount of lactate present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w).

In another preferred embodiment, the composition of the invention comprises urea as a nitrogen source. Urea is a natural moisturizing factor of the skin which has moisture-binding properties. Urea binds moisture in the upper skin layer and helps to regulate the moisture balance. It is preferred that the urea is present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.7 to 2.0% (w/w), more preferably 0.8 to 1.5% (w/w), such as 1.0% (w/w). In some embodiments, the amount of urea present in the composition of the invention is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w).

In another preferred embodiment, the composition of the invention comprises arginine as a nitrogen source. The arginine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of arginine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w).

In another preferred embodiment, the composition of the invention comprises caprylic/capric triglycerides as a lipid source. Caprylic/Capric triglycerides are medium-chain fatty acids which are also referred to as neutral oil or MCT oil. In cosmetic products, caprylic/capric triglycerides are often used to improve the application behaviour of the product and to prevent excessive refatting. The caprylic/capric triglycerides are preferably present an amount of 0.1 to 6.0% (w/w), more preferably 0.2 to 6.0% (w/w), more preferably 0.3 to 5.5% (w/w), more preferably 0.4 to 5.0% (w/w), more preferably 0.5 to 4.5% (w/w), more preferably 0.6 to 4.0% (w/w), more preferably 0.7 to 3.5% (w/w), more preferably 0.8 to 3.0% (w/w), more preferably 0.9 to 2.5% (w/w), more preferably 1.0 to 3.0% (w/w), more preferably 1.5 to 2.5% (w/w), such as 2.0% (w/w). In some embodiments, the amount of caprylic/capric triglycerides present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), at least 3.0 (w/w), at least 4.0 (w/w), or at least 5.0 (w/w).

A particularly preferred skin care composition of the invention has an osmolality of 4000 mOsm/kg water or less and comprises

(a) at least 50% (w/w) water,
(b) less than 0.5% (w/w) of NaCl,
(c) less than 5% (w/w) of glycerol, and
(d) 0.05 to 5.0% (w/w) pyruvate as a carbon source.

Another particularly preferred skin care composition of the invention has an osmolality of 4000 mOsm/kg water or less and comprises

(a) at least 50% (w/w) water,

(b) less than 0.5% (w/w) of NaCl,
(c) less than 5% (w/w) of glycerol, and
(d) 0.05 to 5.0% (w/w) lactate as a carbon source.

Another particularly preferred skin care composition of the invention has an osmolality of 4000 mOsm/kg water or less and comprises

(a) at least 50% (w/w) water,
(b) less than 0.5% (w/w) of NaCl,
(c) less than 5% (w/w) of glycerol, and
(d) 0.05 to 5.0% (w/w) urea as a nitrogen source.

Another particularly preferred skin care composition of the invention has an osmolality of 4000 mOsm/kg water or less and comprises

(a) at least 50% (w/w) water,
(b) less than 0.5% (w/w) of NaCl,
(c) less than 5% (w/w) of glycerol, and
(d) 0.05 to 5.0% (w/w) arginine as a nitrogen source.

Another particularly preferred skin care composition of the invention has an osmolality of 4000 mOsm/kg water or less and comprises

(a) at least 50% (w/w) water,
(b) less than 0.5% (w/w) of NaCl,
(c) less than 5% (w/w) of glycerol, and
(d) 0.1 to 6.0% (w/w) caprylic/capric triglycerides as a lipid source.

Another particularly preferred skin care composition of the invention has an osmolality of 3800 mOsm/kg water or less and comprises

(a) at least 70% (w/w) water,
(b) less than 0.5% (w/w) of NaCl,
(c) less than 5% (w/w) of glycerol, and
(d) 0.05 to 5.0% (w/w) pyruvate as a carbon source.

Another particularly preferred skin care composition of the invention has an osmolality of 3800 mOsm/kg water or less and comprises

(a) at least 70% (w/w) water,
(b) less than 0.5% (w/w) of NaCl,
(c) less than 5% (w/w) of glycerol, and
(d) 0.05 to 5.0% (w/w) lactate as a carbon source.

Another particularly preferred skin care composition of the invention has an osmolality of 3800 mOsm/kg water or less and comprises

(a) at least 70% (w/w) water,
(b) less than 0.5% (w/w) of NaCl,
(c) less than 5% (w/w) of glycerol, and
(d) 0.05 to 5.0% (w/w) urea as a nitrogen source.

Another particularly preferred skin care composition of the invention has an osmolality of 3800 mOsm/kg water or less and comprises

(a) at least 70% (w/w) water,
(b) less than 0.5% (w/w) of NaCl,
(c) less than 5% (w/w) of glycerol, and

(d) 0.05 to 5.0% (w/w) arginine as a nitrogen source.

Another particularly preferred skin care composition of the invention has an osmolality of 3800 mOsm/kg water or less and comprises

(a) at least 70% (w/w) water,
(b) less than 0.5% (w/w) of NaCl,
(c) less than 5% (w/w) of glycerol, and
(d) 0.1 to 6.0% (w/w) caprylic/capric triglycerides as a lipid source.

Another particularly preferred skin care composition of the invention has an osmolality of 4000 mOsm/kg water or less and comprises

(a) at least 50% (w/w) water,
(b) less than 0.1% (w/w) of NaCl,
(c) less than 5% (w/w) of glycerol, and
(d) 0.05 to 5.0% (w/w) pyruvate as a carbon source.

Another particularly preferred skin care composition of the invention has an osmolality of 4000 mOsm/kg water or less and comprises

(a) at least 50% (w/w) water,
(b) less than 0.1% (w/w) of NaCl,
(c) less than 5% (w/w) of glycerol, and
(d) 0.05 to 5.0% (w/w) lactate as a carbon source.

Another particularly preferred skin care composition of the invention has an osmolality of 4000 mOsm/kg water or less and comprises

(a) at least 50% (w/w) water,
(b) less than 0.1% (w/w) of NaCl,
(c) less than 5% (w/w) of glycerol, and
(d) 0.05 to 5.0% (w/w) urea as a nitrogen source.

Another particularly preferred skin care composition of the invention has an osmolality of 4000 mOsm/kg water or less and comprises

(a) at least 50% (w/w) water,
(b) less than 0.1% (w/w) of NaCl,
(c) less than 5% (w/w) of glycerol, and
(d) 0.05 to 5.0% (w/w) arginine as a nitrogen source.

Another particularly preferred skin care composition of the invention has an osmolality of 4000 mOsm/kg water or less and comprises

(a) at least 50% (w/w) water,
(b) less than 0.1% (w/w) of NaCl,
(c) less than 5% (w/w) of glycerol, and
(d) 0.1 to 6.0% (w/w) caprylic/capric triglycerides as a lipid source.

Another particularly preferred skin care composition of the invention has an osmolality of 4000 mOsm/kg water or less and comprises

(a) at least 50% (w/w) water,
(b) less than 0.1% (w/w) of NaCl,
(c) less than 0.1% (w/w) of glycerol, and
(d) 0.05 to 5.0% (w/w) pyruvate as a carbon source.

Another particularly preferred skin care composition of the invention has an osmolality of 4000 mOsm/kg water or less and comprises

(a) at least 50% (w/w) water,
(b) less than 0.1% (w/w) of NaCl,
(c) less than 0.1% (w/w) of glycerol, and
(d) 0.05 to 5.0% (w/w) lactate as a carbon source.

Another particularly preferred skin care composition of the invention has an osmolality of 4000 mOsm/kg water or less and comprises

(a) at least 50% (w/w) water,
(b) less than 0.1% (w/w) of NaCl,
(c) less than 0.1% (w/w) of glycerol, and
(d) 0.05 to 5.0% (w/w) urea as a nitrogen source.

Another particularly preferred skin care composition of the invention has an osmolality of 4000 mOsm/kg water or less and comprises

(a) at least 50% (w/w) water,
(b) less than 0.1% (w/w) of NaCl,
(c) less than 0.1% (w/w) of glycerol, and
(d) 0.05 to 5.0% (w/w) arginine as a nitrogen source.

Another particularly preferred skin care composition of the invention has an osmolality of 4000 mOsm/kg water or less and comprises

(a) at least 50% (w/w) water,
(b) less than 0.1% (w/w) of NaCl,
(c) less than 0.1% (w/w) of glycerol, and
(d) 0.1 to 6.0% (w/w) caprylic/capric triglycerides as a lipid source.

Another particularly preferred skin care composition of the invention has an osmolality of 3800 mOsm/kg water or less and comprises

(a) at least 70% (w/w) water,
(b) no NaCl,
(c) no glycerol, and
(d) 0.05 to 5.0% (w/w) pyruvate as a carbon source.

Another particularly preferred skin care composition of the invention has an osmolality of 3800 mOsm/kg water or less and comprises

(a) at least 70% (w/w) water,
(b) no NaCl,
(c) no glycerol, and
(d) 0.05 to 5.0% (w/w) lactate as a carbon source.

Another particularly preferred skin care composition of the invention has an osmolality of 3800 mOsm/kg water or less and comprises

(a) at least 70% (w/w) water,
(b) no NaCl,
(c) no glycerol, and
(d) 0.05 to 5.0% (w/w) urea as a nitrogen source.

Another particularly preferred skin care composition of the invention has an osmolality of 4000 mOsm/kg water or less and comprises

(a) at least 70% (w/w) water,
(b) no NaCl,
(c) no glycerol, and
(d) 0.05 to 5.0% (w/w) arginine as a nitrogen source.

Another particularly preferred skin care composition of the invention has an osmolality of 4000 mOsm/kg water or less and comprises

(a) at least 70% (w/w) water,
(b) no NaCl,
(c) no glycerol, and
(d) 0.1 to 6.0% (w/w) caprylic/capric triglycerides as a lipid source.

The skin care composition of the present invention can further comprise additional compounds which are normally present in topical skin compositions. For example, the skin care composition of the present invention can further comprise at least one compound selected from the group consisting of perfumes, emollients, pigments, thickener, fillers, colorants, antioxidants, surfactants, lubricants, stabilizers, preservatives, solubilizers, emulsifiers and combinations of any of these.

The skin care compositions described herein are particularly useful for maintaining or promoting the vitality of bacteria of the skin microbiome. The bacteria can be the resident bacteria of the skin microbiome. Alternative, the bacteria can be bacteria that are applied to the skin surface to support and supplement the microbiome. The compositions preferably maintain the overall number of microorganisms, more preferably the overall number of bacteria, in a skin areal to which the skin care compositions are applied. It is particularly preferred that the overall number of microorganisms, more preferably the overall number of bacteria, is reduced after daily topical application of the composition over a period of 2-4 weeks by not more than 25%, not more than 20%, not more than 15%, not more than 10%, not more than 7.5%, or not more than 5% relative to an untreated areal of the part of the body. It is particularly preferred that the number of bacteria of the species *S. epidermidis* is essentially maintained after daily topical application of the composition over a period of 2-4 weeks. Preferably, the number of *S. epidermidis* is reduced after daily topical application of the composition over a period of 2-4 weeks by not more than 25%, not more than 20%, not more than 15%, not more than 10%, not more than 7.5%, or not more than 5% relative to an untreated areal of the part of the body.

The compositions of the invention are useful for both cosmetic and therapeutic purposes, in particular for preventing or treating skin diseases like acne. Hence, in another aspect the invention provides a skin care composition as defined hereinabove for use in a method of treating a skin disease, preferably acne. In one embodiment, the skin care composition is used for preventing the formation of acne. In another embodiment, the skin care composition is used for treating an acute state of acne. In yet preferred embodiment, the skin care composition is used for preventing the reoccurrence of acne in a subject who has received a standard acne treatment. It is particularly preferred that the subject is a human.

The invention also provides methods for treating the skin of a subject by administering a skin care composition as described hereinabove. In one aspect, a method of treating a skin disease, preferably acne, is provided, said method comprising the topical administration of a skin care composition as described hereinabove.

Apart from therapeutic uses, the compositions of the invention can also be used for non-therapeutic, cosmetic purposes, e.g. for promoting the development of or stabilizing a healthy skin microbiome, for improving the appearance of the skin of a subject, for maintaining healthy skin, or for preventing a deterioration of the skin microbiome. Accordingly, the present invention also relates to the use, preferably, the non-therapeutic use, of a water-containing skin care composition which

(a) is formulated for topical application to the skin, and

(b) has an osmolality of 4000 mOsm/kg water or less,

for maintaining or promoting the vitality of bacteria on the skin surface, for promoting the development of or maintaining a healthy skin microbiome. In one embodiment, the composition comprises less than 0.5% (w/w) of NaCl, preferably less than 0.1% (w/w) of NaCl. In one embodiment, the composition comprises less than 5% (w/w) of glycerol, preferably less than 1% (w/w) of glycerol. It is particularly preferred that a composition having an osmolality of 4000 mOsm/kg water and containing less than 0.5% (w/w) of NaCl and less than 5% (w/w) of glycerol is used for maintaining or promoting the vitality of bacteria on the skin surface. It is also particularly preferred that a composition having an osmolality of 4000 mOsm/kg water and containing less than 0.1% (w/w) of NaCl and less than 1% (w/w) of glycerol is used for maintaining or promoting the vitality of bacteria on the skin surface.

**BRIEF DESCRIPTION OF THE FIGURE**

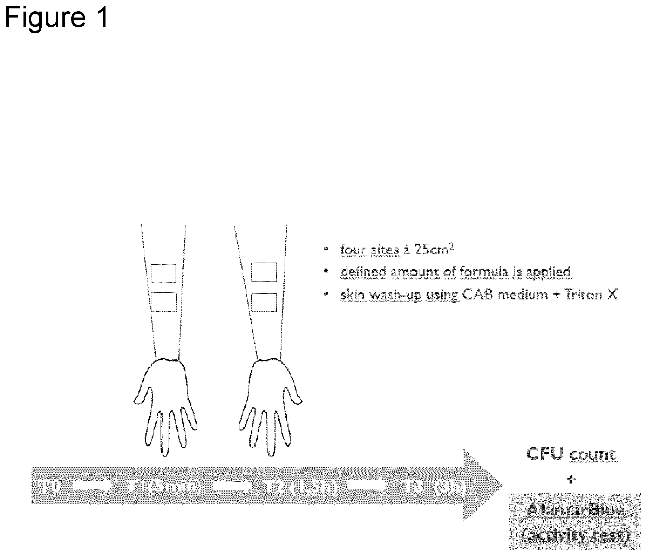

Figure 1 shows the general procedure of the AlamarBlue Forearm Test to measure the viability of the bacterial cells on the skin surface in response to the test formulas.

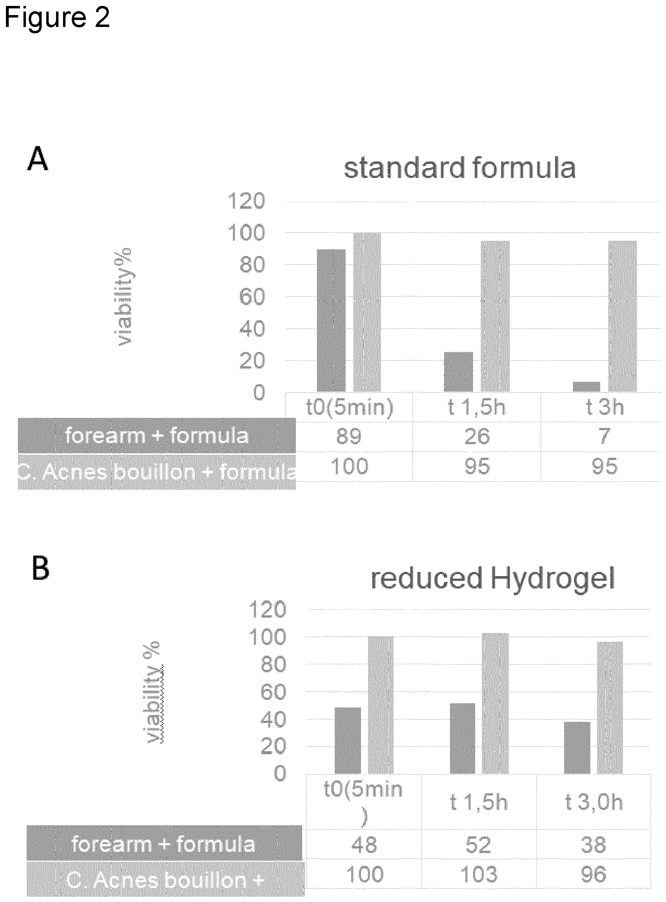

Figure 2 is a bar diagram showing the results of the AlamarBlue Forearm Test to measure the viability of the bacterial cells on the skin surface in response to the test formulas. It can be seen that hydrogels having a reduced osmolality maintain viability of the bacterial cells much better compared to a standard hydrogel.

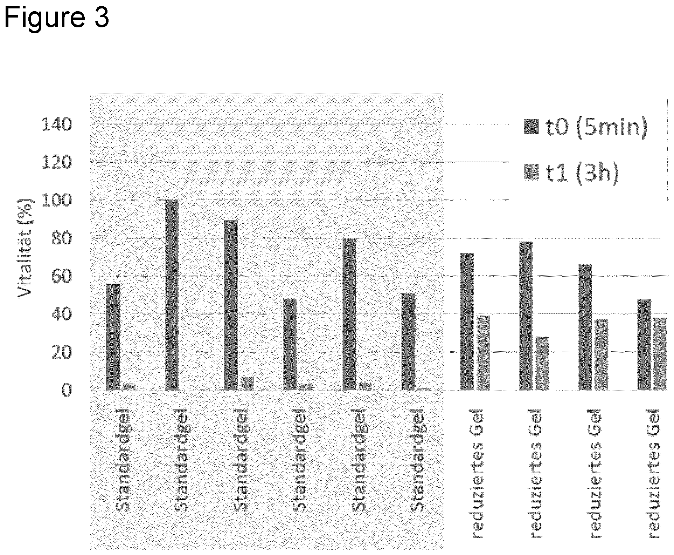

Figure 3 is a bar diagram showing the results of the AlamarBlue Forearm Test to measure the viability of the bacterial cells on the skin surface in response to the test formulas. It can be seen that hydrogels having a reduced osmolality (n=4) maintain viability of the bacterial cells much better compared to a standard hydrogel (n=6).

**EXAMPLES**

The present invention is further illustrated by the following examples, which in no way should be construed as limiting. The entire contents of all of the references (including literature references, issued patents, published patent applications, and co pending patent applications) cited throughout this application are hereby expressly incorporated by reference.

Example 1: Formulation of Standard and test gels

The following 4 standard gels were formulated. The amounts of osmotically active compounds in the gels were selected as commonly used in the field. The theoretical osmolality of the compositions was calculated.

Standard Gel 1:

| | g | per kg | Molar mass | Mol/kg Water | Number particles /ions | Osmolality Mol particles/kg Water |
|---|---|---|---|---|---|---|
| Perfume | 0.135 | 1.35 | | | | |
| Glycerine | 7.5 | 75 | 92,09 | 1.040288008 | 1 | 1.040 |
| Citric acid | 0.02 | 0.2 | 210,03 | 0.001216336 | 3 | 0.003649007 |
| Sodium Citrate | 0.174 | 1.74 | 215,03 | 0.010336059 | 4 | 0.041344237 |
| Caustic Soda | 0.017 | 0.17 | 40 | 0.005428674 | 2 | 0.010857347 |
| Ethanol | 11.466 | 114.66 | 46,07 | 3.179058552 | 1 | 3.179058552 |
| Sodium Chloride | 0,9 | 9 | 58,5 | 0.196513072 | 2 | 0.393026144 |
| Magnesium Chloride | 0 | 0 | 95,21 | 0 | 3 | 0 |
| Magnesium Sulfate | 0 | 0 | 120,37 | 0 | 2 | 0 |
| Carbomer | 1 | 10 | | | | 0 |
| Sodium Hyaluronate | 0 | 0 | | | | 0 |
| PEG-40 Hydrogen-ated Castor Oil | 0,5 | 5 | 939.5 | 0.006797951 | 1 | 0.006797951 |
| Water | 78.28800 | 782.88 | | | | 0 |
| Total: | 100 | 1000 | | | | 4.675 |

Standard Gel 2:

|  | g | per kg | Molar mass | Mol/kg Water | Number particles /ions | Osmolality Mol particles/kg Water |
|---|---|---|---|---|---|---|
| Perfume | 0.135 | 1.35 |  |  |  |  |
| Glycerine | 7.5 | 75 | 92.09 | 1.036976591 | 1 | 1.037 |
| Citric acid | 0.02 | 0.2 | 210.03 | 0.001212464 | 3 | 0.003637392 |
| Sodium Citrate | 0.174 | 1.74 | 215.03 | 0.010303158 | 4 | 0.041212632 |
| Caustic Soda | 0.017 | 0.17 | 40 | 0.005411393 | 2 | 0.010822786 |
| Ethanol | 11.466 | 114.66 | 46.07 | 3.16893906 | 1 | 3.16893906 |
| Sodium Chloride | 0.9 | 9 | 58.5 | 0.195887537 | 2 | 0.391775074 |
| Magnesium Chloride | 0 | 0 | 95.21 | 0 | 3 | 0 |
| Magnesium Sulfate | 0 | 0 | 120.37 | 0 | 2 | 0 |
| Carbomer | 0 | 0 |  |  |  | 0 |
| Sodium Hyaluronate | 0.75 | 7.5 |  |  |  | 0 |
| PEG-40 Hydrogenated Castor Oil | 0.5 | 5 | 939.5 | 0.006776312 | 1 | 0.006776312 |
| Water | 78.53800 | 785.38 |  |  |  |  |
| Total: | 100 | 1000 |  |  |  | 4.660 |

Standard Gel 3:

|  | g | per kg | Molar mass | Mol/kg Water | Number particles /ions | Osmolality Mol particles/kg Water |
|---|---|---|---|---|---|---|
| Perfume | 0.135 | 1.35 |  |  |  |  |
| Glycerine | 7.5 | 75 | 92.09 | 1.036976591 | 1 | 1.037 |
| Citric acid | 0.02 | 0.2 | 210.03 | 0.001212464 | 3 | 0.003637392 |
| Sodium Citrate | 0.174 | 1.74 | 215.03 | 0.010303158 | 4 | 0.041212632 |
| Caustic Soda | 0.017 | 0.17 | 40 | 0.005411393 | 2 | 0.010822786 |
| Ethanol | 11.466 | 114.66 | 46.07 | 3.16893906 | 1 | 3.16893906 |
| Sodium Chloride | 0 | 0 | 58.5 | 0 | 2 | 0 |
| Magnesium Chloride | 0.9 | 9 | 95.21 | 0.120359426 | 3 | 0.361078277 |
| Magnesium Sulfate | 0 | 0 | 120.37 |  | 2 | 0 |
| Carbomer | 0 | 0 |  |  |  | 0 |
| Sodium Hyaluronate | 0.75 | 7.5 |  |  |  | 0 |
| PEG-40 Hydrogenated Castor Oil | 0.5 | 5 | 939.5 | 0.006776312 | 1 | 0.006776312 |
| Water | 78.53800 | 785.38 |  |  |  | 0 |
| Total: | 100 | 1000 |  |  |  | 4.629 |

Standard Gel 4:

| | g | per kg | Molar mass | Mol/kg Water | Number particles /ions | Osmolality Mol particles/kg Water |
|---|---|---|---|---|---|---|
| Perfume | 0.135 | 1.35 | | | | |
| Glycerine | 7.5 | 75 | 92.09 | 1.040288008 | 1 | 1.040 |
| Citric acid | 0.02 | 0.2 | 210.03 | 0.001216336 | 3 | 0.003649007 |
| Sodium Citrate | 0.174 | 1.74 | 215.03 | 0.010336059 | 4 | 0.041344237 |
| Caustic Soda | 0.017 | 0.17 | 40 | 0.005428674 | 2 | 0.010857347 |
| Ethanol | 11.466 | 114.66 | 46.07 | 3.179058552 | 1 | 3.179058552 |
| Sodium Chloride | 0 | 0 | 58.5 | 0 | 2 | 0 |
| Magnesium Chloride | 0 | 0 | 95.21 | 0 | 3 | 0 |
| Magnesium Sulfate | 0.9 | 9 | 120.37 | 0.095505647 | 2 | 0.191011294 |
| Carbomer | 1 | 10 | | | | 0 |
| Sodium Hyaluronate | 0 | 0 | | | | 0 |
| PEG-40 Hydrogenated Castor Oil | 0.5 | 5 | 939.5 | 0.006797951 | 1 | 0.006797951 |
| Water | 78.28800 | 782.88 | | | | 0 |
| Total: | 100 | 1000 | | | | 4.473 |

In addition, the following 4 gels with reduced osmolality were formulated. The amounts of osmotically active compounds in these gels were selected to be lower than the amounts commonly used in the field. The theoretical osmolality of the compositions was calculated.

Test Gel 1 with reduced osmolality:

| | g | per kg | Molar mass | Mol/kg Water | Number particles /ions | Osmolality Mol particles/kg Water |
|---|---|---|---|---|---|---|
| Perfume | 0.135 | 1.35 | | | | |
| Glycerine | 2.5 | 25 | 92.09 | 0.321735382 | 1 | 0.322 |
| Citric acid | 0.002 | 0.02 | 210.03 | 0.000112855 | 3 | 0.000338564 |
| Sodium Citrate | 0.0174 | 0.174 | 215.03 | 0.000959006 | 4 | 0.003836025 |
| Caustic Soda | 0.0017 | 0.017 | 40 | 0.000503686 | 2 | 0.001007373 |
| Ethanol | 11.466 | 114.66 | 46.07 | 2.949612824 | 1 | 2.949612824 |
| Sodium Chloride | 0 | 0 | 58.5 | 0 | 2 | 0 |
| Magnesium Chloride | 0 | 0 | 95.21 | 0 | 3 | 0 |
| Magnesium Sulfate | 0 | 0 | 120.37 | 0 | 2 | 0 |
| Carbomer | 1 | 10 | | | | 0 |
| Sodium Hyaluronate | 0 | 0 | | | | 0 |
| PEG-40 Hydrogenated Castor Oil | 0.5 | 5 | 939.5 | 0.006307315 | 1 | 0.006307315 |
| Water | 84.37790 | 843.779 | | | | 0 |
| Total: | 100 | 1000 | | | | 3.283 |

Test Gel 2 with reduced osmolality:

| | g | per kg | Molar mass | Mol/kg Water | Number particles /ions | Osmolality Mol particles/kg Water |
|---|---|---|---|---|---|---|
| Perfume | 0.135 | 1.35 | | | | |
| Glycerine | 2.5 | 25 | 92.09 | 0.32078494 | 1 | 0.321 |
| Citric acid | 0.002 | 0.02 | 210.03 | 0.000112521 | 3 | 0.000337564 |
| Sodium Citrate | 0.0174 | 0.174 | 215.03 | 0.000956173 | 4 | 0.003824693 |
| Caustic Soda | 0.0017 | 0.017 | 40 | 0.000502198 | 2 | 0.001004397 |
| Ethanol | 11.466 | 114.66 | 46.07 | 2.940899347 | 1 | 2.940899347 |
| Sodium Chloride | 0 | 0 | 58.5 | 0 | 2 | 0 |
| Magnesium Chloride | 0 | 0 | 95.21 | 0 | 3 | 0 |
| Magnesium Sulfate | 0 | 0 | 120.37 | 0 | 2 | 0 |
| Carbomer | 0 | 0 | | | | 0 |
| Sodium Hyaluronate | 0.75 | 7.5 | | | | 0 |
| PEG-40 Hydrogen-ated Castor Oil | 0.5 | 5 | 939.5 | 0.006288682 | 1 | 0.006288682 |
| Water | 84.62790 | 846.279 | | | | 0 |
| Total: | 100 | 1000 | | | | 3.273 |

Test Gel 3 with reduced osmolality:

| | g | per kg | Molar mass | Mol/kg Water | Number particles /ions | Osmolality Mol particles/kg Water |
|---|---|---|---|---|---|---|
| Perfume | 0.135 | 1.35 | | | | |
| Glycerine | 1 | 10 | 92.09 | 0.126446296 | 1 | 0.126 |
| Citric acid | 0.002 | 0.02 | 210.03 | 0.000110884 | 3 | 0.000332651 |
| Sodium Citrate | 0.0174 | 0.174 | 215.03 | 0.000942256 | 4 | 0.003769023 |
| Caustic Soda | 0.0017 | 0.017 | 40 | 0.000494889 | 2 | 0.000989777 |
| Ethanol | 11.466 | 114.66 | 46.07 | 2.898092942 | 1 | 2.898092942 |
| Sodium Chloride | 0 | 0 | 58.5 | 0 | 2 | 0 |
| Magnesium Chloride | 0 | 0 | 95.21 | 0 | 3 | 0 |
| Magensium Sulfate | 0 | 0 | 120.37 | 0 | 2 | 0 |
| Carbomer | 1 | 10 | | | | 0 |
| Sodium Hyaluronate | 0 | 0 | | | | 0 |
| PEG-40 Hydrogen-ated Castor Oil | 0.5 | 5 | 939.5 | 0.006197147 | 1 | 0.006197147 |
| Water | 85.87790 | 858.779 | | | | 0 |
| Total: | 100 | 1000 | | | | 3.036 |

Test Gel 4 with reduced osmolality:

| | g | per kg | Molar mass | Mol/kg Water | Number particles /ions | Osmolality |
|---|---|---|---|---|---|---|
| | | | | | | Mol particles/kg Water |
| Perfume | 0 | 0 | | | | |
| Glycerine | 2.5 | 25 | 92.09 | 0.320274033 | 1 | 0.320 |
| Citric acid | 0.002 | 0.02 | 210.03 | 0.000112342 | 3 | 0.000337027 |
| Sodium Citrate | 0.0174 | 0.174 | 215.03 | 0.00095465 | 4 | 0.003818602 |
| Caustic Soda | 0.0017 | 0.017 | 40 | 0.000501399 | 2 | 0.001002797 |
| Ethanol | 11.466 | 114.66 | 46.07 | 2.936215442 | 1 | 2.936215442 |
| Sodium Chloride | 0 | 0 | 58.5 | 0 | 2 | 0 |
| Magnesium Chloride | 0 | 0 | 95.21 | 0 | 3 | 0 |
| Magnesium Sulfate | 0 | 0 | 120.37 | 0 | 2 | 0 |
| Carbomer | 0 | 0 | | | | 0 |
| Sodium Hyaluronate | 0.75 | 7.5 | | | | 0 |
| PEG-40 Hydrogenated Castor Oil | 0.5 | 5 | 939.5 | 0.006278666 | 1 | 0.006278666 |
| Water | 84.76290 | 847.629 | | | | 0 |
| Total: | 100 | 1000 | | | | 3.268 |

Example 2: AlamarBlue Forearm Test

Before the test is performed, 4 areas of 25 cm$^2$ to be tested are marked on the forearm of the test subjects. Then, a defined amount of the respective formulas are applied leaving one test area untreated for control. At the respective points in time (T0 - 5 min after application, T1 - after 1.5 h, T2 - after 3 h) skin rinsing samples are taken using the swab technique. After sampling, the swab remains in the rinsing solution. As a reference for comparison, the respective formula is pipetted into bacterial medium (CAB) and also incubated for the appropriate time. Then the sample and the reference are diluted and mixed with the AlamarBlue reagent. A triple determination is performed in each case. The test plate is incubated at 37°C for 3 hours and then the fluorescence (RLU) is determined in the plate reader. The steps of the test are depicted in Figure 1.

Results: The results of the AlamarBlue forearm test for Standard Gel 2 and the reduced Test Gel 2 (with reduced osmolality) are depicted in Figure 2 and 3. A significant decrease in the viability of the resident skin bacteria was observed after use of the standard formula. In contrast, the reduced formula was more suited to maintain viability of the cells 3 h after application of the formulas. Essentially the same difference was observed when testing the other Standard Gels and comparing them to the Test Gels.

The effect of osmolality is further enhanced by the ethanol that is present in the gels. After application to the skin, the ethanol and most of the water evaporates which leads to a concentration of the remaining solutes. This results in a significant increase in the osmolality of the Standard Gel. In contrast, as shown below, the osmolality of the Test Gel 2 was low also after ethanol evaporation, therefore imposing less stress on the skin bacteria.

Standard Gel 2 after ethanol evaporation:

| | g | per kg | Molar mass | Mol/kg Water | Number particles /ions | Osmolality Mol particles/kg Water |
|---|---|---|---|---|---|---|
| Perfume | 0,135 | 1,35 | | | | |
| Glycerine | 7,5 | 75 | 92.09 | 9,048716451 | 1 | 9,049 |
| Citric acid | 0,02 | 0,2 | 210.03 | 0,010580029 | 3 | 0,031740087 |
| Sodium Citrate | 0,174 | 1,74 | 215.03 | 0,089905939 | 4 | 0,359623757 |
| Caustic Soda | 0,017 | 0,17 | 40 | 0,047220124 | 2 | 0,094440247 |

| | g | per kg | Molar mass | Mol/kg Water | Number particles /ions | Osmolality Mol particles/kg Water |
|---|---|---|---|---|---|---|
| Ethanol | 0 | 0 | 46.07 | 0 | 1 | 0 |
| Sodium Chloride | 0,9 | 9 | 58.5 | 1,709325739 | 2 | 3,418651479 |
| Magnesium Chloride | 0 | 0 | 95.21 | 0 | 3 | |
| Magnesium Sulfate | 0 | 0 | 120.37 | 0 | 2 | |
| Carbomer | 0 | 0 | | | | |
| Sodium Hyaluronate | 0,75 | 7,5 | | | | |
| PEG-40 Hydrogenated Castor Oil | 0,5 | 5 | 939.5 | 0,059130481 | 1 | 0,059130481 |
| Water | 9,00040 | 90,004 | | | | |
| Total: | 18,9964 | 189,964 | | | | 13,012 |

Test Gel 2 after ethanol evaporation:

| | g | per kg | Molar mass | Mol/kg Water | Number particles /ions | Osmolality Mol particles/kg Water |
|---|---|---|---|---|---|---|
| Perfume | 0,135 | 1,35 | | | | |
| Glycerine | 2,5 | 25 | 92.09 | 2,82508628 | 1 | 2,825 |
| Citric acid | 0,002 | 0,02 | 210.03 | 0,000990953 | 3 | 0,002972858 |
| Sodium Citrate | 0,0174 | 0,174 | 215.03 | 0,00842082 | 4 | 0,033683279 |
| Caustic Soda | 0,0017 | 0,017 | 40 | 0,004422757 | 2 | 0,008845515 |
| Ethanol | 0 | 0 | 46.07 | 0 | 1 | 0 |
| Sodium Chloride | 0 | 0 | 58.5 | 0 | 2 | 0 |
| Magnesium Chloride | 0 | 0 | 95.21 | 0 | 3 | 0 |
| Magnesium Sulfate | 0 | 0 | 120.37 | 0 | 2 | 0 |
| Carbomer | 0 | 0 | | | | |
| Sodium Hyaluronate | 0,75 | 7,5 | | | | |
| PEG-40 Hydrogenated Castor Oil | 0,5 | 5 | 939.5 | 0,055383118 | 1 | 0,055383118 |
| Water | 9,60939 | 96,0939 | | | | |
| Total: | 13,51549 | 135,155 | | | | 2,926 |

Claims

1. Skin care composition for maintaining or promoting the vitality of bacteria on the skin surface, wherein said composition

(a) is a water-containing composition which is formulated for topical application to the skin, and
(b) has an osmolality of 4000 mOsm/kg water or less.

2. Skin care composition for maintaining or promoting the vitality of bacteria on the skin surface of claim 1, wherein said osmolality is 3800 mOsm/kg water or less.

3. Skin care composition for maintaining or promoting the vitality of bacteria on the skin surface of claim 2, wherein said osmolality is 3500 mOsm/kg water or less.

4. Skin care composition for maintaining or promoting the vitality of bacteria on the skin surface of any of claims 1-3, wherein said composition comprises at least 30% water (w/w).

5. Skin care composition for maintaining or promoting the vitality of bacteria on the skin surface of claim 4, wherein said composition comprises at least 50% water (w/w).

6. Skin care composition for maintaining or promoting the vitality of bacteria on the skin surface of claim 5, wherein said composition comprises at least 70% water (w/w).

7. Skin care composition for maintaining or promoting the vitality of bacteria on the skin surface of any of claims 1-6, wherein said composition is a gel.

8. Skin care composition for maintaining or promoting the vitality of bacteria on the skin surface of any of claims 1-7, wherein said composition comprises less than 0.5% (w/w) of NaCl.

9. Skin care composition for maintaining or promoting the vitality of bacteria on the skin surface of any of claims 1-7, wherein said composition comprises less than 5% (w/w) of glycerol.

10. Skin care composition for promoting the vitality of bacteria on the skin surface according to any of claims 1-9, further comprising at least one compound selected from the group consisting of a perfume, an emollient, a pigment, a thickener, a filler, a colorant, an antioxidant, a surfactant, a lubricant, a stabilizer, a preservative, a solubilizer, an emulsifier or a combination of any of these.

11. Skin care composition for promoting the vitality of bacteria on the skin surface according to any of claims 1-10 for use in a method of treating a skin disease, preferably acne.

12. Non-therapeutic use of a water-containing composition which is formulated for topical application to the skin and has an osmolality of 4000 mOsm/kg water or less for promoting the vitality of bacteria on the skin surface.

13. Non-therapeutic use of claim 12, wherein said composition comprises less than 0.5% (w/w) of NaCl.

14. Non-therapeutic use of claim 13, wherein said composition comprises less than 5% (w/w) of glycerol.

15. Non-therapeutic use of any of claims 12-14, wherein said composition comprises less than 0.1% (w/w) of NaCl and less than 1% (w/w) of glycerol.

# Figure 1

- four sites á 25cm$^2$
- defined amount of formula is applied
- skin wash-up using CAB medium + Triton X

T0 ➡ T1 (5min) ➡ T2 (1,5h) ➡ T3 (3h)

CFU count
+
AlamarBlue
(activity test)

# Figure 2

## A

### standard formula

| | t0(5min) | t 1,5h | t 3h |
|---|---|---|---|
| forearm + formula | 89 | 26 | 7 |
| C. Acnes bouillon + formula | 100 | 95 | 95 |

## B

### reduced Hydrogel

| | t0(5min) | t 1,5h | t 3,0h |
|---|---|---|---|
| forearm + formula | 48 | 52 | 38 |
| C. Acnes bouillon + | 100 | 103 | 96 |

# Figure 3

Europäisches
Patentamt

European
Patent Office

Office européen
des brevets

# EUROPEAN SEARCH REPORT

**Application Number**

EP 20 19 7603

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/173056 A1 (EBERTING CHERYL LEE [US]) 22 June 2017 (2017-06-22) | 1-15 | INV. A61Q19/00 |
| Y | * claims 1,6,12,13,14,26,27 * <br> * paragraph [0002] * <br> * page 10; examples B,C * | 1-15 | A61P17/00 <br> A61P17/10 <br> A61K8/34 <br> A61K8/365 |
| X,D | WO 2019/238968 A1 (BEIERSDORF AG [DE]; S BIOMEDIC [BE]) 19 December 2019 (2019-12-19) | 1-15 | A61K8/19 <br> A61K8/81 <br> A61K8/73 |
| Y | * claims 1,8,21,23 * <br> * page 2, paragraph 4 * <br> * page 10; examples 1-4 * | 1-15 | A61K8/86 |
| X | WO 2019/096635 A1 (DSM IP ASSETS BV [NL]) 23 May 2019 (2019-05-23) | 1-14 | |
| Y | * claims 7-11 * <br> * page 2, lines 14-17 * <br> * page 3, lines 9-12 * <br> * page 17; table 2 * | 1-15 | |
| X | DE 101 33 202 A1 (BEIERSDORF AG [DE]) 16 January 2003 (2003-01-16) | 1-15 | **TECHNICAL FIELDS SEARCHED (IPC)** |
| Y | * claims * <br> * paragraphs [0022] - [0023] * <br> * column 20; examples 3,4 * | 1-15 | A61P <br> A61Q <br> A61K |
| X | WO 2009/061431 A2 (ACIEX INC [US]; CHAPIN MATTHEW JONATHAN [US] ET AL.) 14 May 2009 (2009-05-14) | 1-6, 10-12 | |
| Y | * claims 1,6 * <br> * pages 40-41; example 10 * <br> * figures 18A,18B,19 * | 1-15 | |
| X | US 2019/336415 A1 (ADKINS JR NAT [US] ET AL) 7 November 2019 (2019-11-07) | 1-3,10, 11 | |
| Y | * claims 1,11,12,22 * <br> * paragraphs [0006] - [0007] * <br> * paragraph [0028] * | 1-15 | |

The present search report has been drawn up for all claims

| Place of search | Date of completion of the search | Examiner |
|---|---|---|
| Munich | 25 February 2021 | Grillenberger, Sonja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 19 7603

This annex lists the patent family members relating to the patent documents cited in the above-mentioned European search report.
The members are as contained in the European Patent Office EDP file on
The European Patent Office is in no way liable for these particulars which are merely given for the purpose of information.

25-02-2021

| Patent document cited in search report | | Publication date | Patent family member(s) | | Publication date |
|---|---|---|---|---|---|
| US 2017173056 | A1 | 22-06-2017 | CN | 106470677 A | 01-03-2017 |
| | | | EP | 3119387 A1 | 25-01-2017 |
| | | | JP | 2017509705 A | 06-04-2017 |
| | | | US | 2017173056 A1 | 22-06-2017 |
| | | | US | 2019336517 A1 | 07-11-2019 |
| | | | WO | 2015143399 A1 | 24-09-2015 |
| WO 2019238968 | A1 | 19-12-2019 | CA | 3102693 A1 | 19-12-2019 |
| | | | CN | 112292116 A | 29-01-2021 |
| | | | WO | 2019238968 A1 | 19-12-2019 |
| WO 2019096635 | A1 | 23-05-2019 | EP | 3709955 A1 | 23-09-2020 |
| | | | WO | 2019096635 A1 | 23-05-2019 |
| DE 10133202 | A1 | 16-01-2003 | DE | 10133202 A1 | 16-01-2003 |
| | | | EP | 1406588 A2 | 14-04-2004 |
| | | | JP | 2004536838 A | 09-12-2004 |
| | | | US | 2004220137 A1 | 04-11-2004 |
| | | | WO | 03005979 A2 | 23-01-2003 |
| WO 2009061431 | A2 | 14-05-2009 | AU | 2008325214 A1 | 14-05-2009 |
| | | | BR | PI0819105 A2 | 24-09-2019 |
| | | | CA | 2705050 A1 | 14-05-2009 |
| | | | CN | 101977595 A | 16-02-2011 |
| | | | EP | 2219630 A2 | 25-08-2010 |
| | | | JP | 2011503061 A | 27-01-2011 |
| | | | WO | 2009061431 A2 | 14-05-2009 |
| US 2019336415 | A1 | 07-11-2019 | AU | 2019262032 A1 | 07-01-2021 |
| | | | CA | 3099166 A1 | 07-11-2019 |
| | | | EP | 3787641 A1 | 10-03-2021 |
| | | | KR | 20210005936 A | 15-01-2021 |
| | | | SG | 11202010874S A | 27-11-2020 |
| | | | TW | 202002949 A | 16-01-2020 |
| | | | US | 2019336415 A1 | 07-11-2019 |
| | | | WO | 2019213266 A1 | 07-11-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019238968 A